(19) Europäisches Patentamt
European Patent Office
Office européen des brevets

(11) EP 1 660 180 B1

# (12) EUROPEAN PATENT SPECIFICATION

(45) Date of publication and mention of the grant of the patent:
**23.01.2008 Bulletin 2008/04**

(21) Application number: **03818427.1**

(22) Date of filing: **28.08.2003**

(51) Int Cl.:
***A61N 1/37*** *(2006.01)* ***A61N 1/365*** *(2006.01)*

(86) International application number:
**PCT/SE2003/001340**

(87) International publication number:
**WO 2005/021091 (10.03.2005 Gazette 2005/10)**

## (54) MEDICAL IMPLANT FOR EVOKED RESPONSE DETECTION HAVING AN ADAPTIVE DETECTION TIME WINDOW

MEDIZINISCHES IMPLANTAT ZUM NACHWEIS VON EVOZIERTEN POTENTIALEN MIT ADAPTIVEM NACHWEISZEITFENSTER

IMPLANT MEDICAL POUR DETECTION DES POTENTIELS EVOQUES A FENETRE TEMPORELLE DE DETECTION ADAPTATIVE

(84) Designated Contracting States:
**CH DE FR IE IT LI**

(43) Date of publication of application:
**31.05.2006 Bulletin 2006/22**

(73) Proprietor: **St. Jude Medical AB**
**175 84 Järfälla (SE)**

(72) Inventor: **BJÖRLING, Anders**
**SE-175 53 Jarfalla (SE)**

(56) References cited:
**EP-A1- 0 906 768**
**WO-A1-99/65569**
**US-A- 4 858 610**
**US-A- 5 718 720**
**US-A- 5 855 594**
**US-A1- 2003 009 200**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### *Technical Field*

The present invention relates to a medical implant comprising pulse generator means for delivering stimulation pulses to at least one chamber of a patient's heart, an evoked response detector for distinguishing capture from loss of capture from the value of a selected one of a plurality of parameters obtainable from an IEGM signal sensed In an evoked response detection time window following delivery of a stimulation pulse, and a setting means for setting a minimum tolerable difference between values of said selected parameter obtained in case of capture and in case of loss of capture respectively.

### *Background to the Invention*

Implantable pacemakers, which automatically detect capture and minimize pacing energy provide many benefits. The use of minimal pacing energy maximizes device longevity and minimizes the size of the device, and most importantly, automatic output regulation protects the patient from loss of capture caused by a rise in the threshold of stimulation.

For automatic capture a cardiac signal sensed in an evoked response detection time window after each stimulation pulse is analysed to determine whether or not the stimulation pulse captured the heart of a patient. The length of the evoked response detection time window is conventionally fixed. If a shorter evoked response detection time window could be used, a stimulation backup pulse could be delivered quicker, however, the shorter evoked response detection time window the greater risk of inaccurate decisions.

The shortest length of an evoked response detection time window that has a tolerable risk of inaccurate decisions depends on the lead type, the lead position, the evoked response of the patient and the parameter used to distinguish capture from loss of capture. Evoked response detection is the heart of the algorithm of automatic capture and thus very important.

There are mainly three different evoked response detection methods today, namely methods using the parameters maximum signal amplitude, maximum signal slope of the sensed IEGM signal, or area obtained by integrating the sensed IEGM signal over the evoked response detection time window. The value of the measured parameter is compared to a pre-set threshold. Values above the threshold indicate capture and values below the threshold indicate loss of capture. Thus, Boriani et. al. describes in the article titled "Atrial Evoked Response Integral for Automatic Capture Verification In Atrial Pacing", PACE 2003, Vol. 26, Part II, page 1-5, January 2003, the Integral of the atrial evoked response signal as a resource for verification of atrial capture.

US 5 718 720 A discloses a method for adjusting the sensitivity of an electrical evoked response detector in an implantable cardiac stimulator electrically coupled to cardiac tissue, comprising: applying a stimulation pulse to one chamber of the heart, detecting mechanical evoked response, detecting the electrical evoked response with a sensor circuit, adjusting the magnitude of the stimulating pulse until loss of capture is detected with respect to said mechanical response, and adjusting the sensitivity of said sensor circuit to detect a corresponding loss of capture. An associated cardiac stimulator is also described, wherein this technique is used.

US 5 855 594 describes a solution providing a process to verify capture or another desired response, that involves assessing the reliability of a chosen parameter of an evoked response as a reliable indication of the response. A process for testing the efficacy of a sensed parameter as an indication of tissue stimulation, and a system for automatically determining the efficacy of a detected response in tissue as an indication of tissue stimulation are disclosed.

### *The Purpose of the Invention*

The purpose of the present invention is to provide an Improved medical implant which is quick in distinguishing capture from loss of capture and with a tolerable risk of inaccurate decisions.

### *Disclosure* **of** *the Invention*

The above-mentioned purpose is obtained by a medical implant comprising pulse generator means for delivering stimulation pulses to at least one chamber of a patient's heart, an evoked response detector for distinguishing capture from loss of capture from the value of a selected one of a plurality of parameters obtainable from an IEGM signal sensed In an evoked response detection time window following delivery of a stimulation pulse, and a setting means for setting a minimum tolerable difference between values of said selected parameter obtained in case of capture and in case of loss of capture respectively, and having a first calculation means provided to calculate for each of said parameters the length of the evoked response detection time window for which said minimum tolerable difference is obtained, and a first selecting means provided to select that parameter for distinguishing capture and loss of capture for which said minimum tolerable difference is obtained with the shortest evoked response detection time window.

Thus, this medical implant is able to automatically select that parameter for distinguishing capture and loss of capture for which said minimum tolerable difference is obtained with the shortest evoked response detection time window. The only requirements are that the evoked response detector is able to distinguish capture from loss of capture with at least one of the available parameters if an evoked response detection time window of a maximum length Is used, where the maximum length can be very large, e.g. 120 ms, and that a minimum tolerable difference between values of said selected parameter obtained in case of capture and in case of loss of capture, respectively, is set.

According to an advantageous embodiment of the medical Implant according to the present invention, a third calculation means is provided to calculate a matrix or table of said difference for different lengths of the evoked response detection time window and different ones of said parameters for storage for use in later off-line analysis.

According to an advantageous embodiment of the medical implant according to the invention, said setting means is adapted to set said minimum tolerable difference with a safety margin. According to a further advantageous embodiment of the medical implant according to the invention, said minimum tolerable difference is pre-set or programmable.

According to another advantageous embodiment of the medical implant according to the invention, said setting means is adapted to calculate as said difference the slgnal-to-nolse-ratio SNR from the equation

$$\mathrm{SNR} = \left\{ \begin{array}{l} \dfrac{\min(ERM_{\text{capture}}) - \max(ERM_{\text{loss of capture}})}{\max(ERM_{\text{capture}}) - \min(ERM_{\text{loss of capture}})}, \overline{ERM}_{\text{capture}} > \overline{ERM}_{\text{loss of capture}} \\ \\ \dfrac{\max(ERM_{\text{capture}}) - \min(ERM_{\text{loss of capture}})}{\min(ERM_{\text{capture}}) - \max(ERM_{\text{loss of capture}})}, \overline{ERM}_{\text{capture}} < \overline{ERM}_{\text{loss of capture}} \end{array} \right\} \text{Equation[1]}$$

$$\overline{ERM}_{\text{capture}} \equiv \frac{1}{N}\sum_{i=1}^{N} ERM_{\text{capture}}(i)$$

where $ERM_{capture}$ and $ERM_{loss\ of\ capture}$ denote the parameter values obtained in case of capture and loss of capture respectively.

According to yet another advantageous embodiment of the medical implant according to the invention, said parameters include maximum signal amplitude and maximum signal slope of the sensed IEGM signal, and area obtained by integrating the sensed IEGM signal over the evoked response detection time window.

According to an advantageous embodiment of the medical implant according to the invention, a differentiating means is provided to calculate the derivative of the sensed IEGM signal for the determination of the maximum slope.

According to a further advantageous embodiment of the medical implant according to the invention, said pulse generator means are controlled to deliver a stimulation back-up pulse at the end of the evoked response detection time window in response to detected loss of capture.

***Brief Description of the Drawings***

The present invention will now, with the purpose of exemplifying, be described more in detail by way of embodiments and by referring to the appended drawings, wherein: -

Fig. 1 shows a diagram showing a sensed evoked response signal resulting from a stimulation pulse,
Fig. 2 shows schematically a first preferred embodiment of the medical implant according to the present invention,
Fig. 3 shows schematically a second preferred embodiment of the medical implant according to the present invention,
Fig. 4 shows a flow diagram of a procedure performed by the first preferred embodiment of the medical implant according to the present invention, and
Fig. 5 shows a flow diagram of a procedure performed by the second preferred embodiment of the medical implant according to the present invention.

### *Description of Preferred Embodiments*

Fig. 1 shows an evoked response resulting from a stimulation pulse. In the diagram an evoked response 1.1 is shown, followed by a T wave 1.2. Further, an evoked response detection time window 1.3 is shown as a rectangle. The signal sensed in this time window 1.3 is analysed to determine whether or not the stimulation pulse has captured the heart. Herein, the length of the window 1.3 is about 39 ms.

Fig. 2 illustrates schematically a first preferred embodiment of the medical implant according to the present invention. The medical implant is connected to a patient's heart 2.1 and comprises pulse generator means 2.2 for delivering stimulation pulses to at least one chamber of the patient's heart 2.1. The pulse generator means 2.2 are controlled to deliver a stimulation back-up pulse at the end of the evoked response detection time window in response to detected loss of capture. The medical implant also comprises an evoked response detector 2.3 for distinguishing capture from loss of capture from the value of a selected one of a plurality of parameters obtainable from an IEGM signal sensed in an evoked response detection time window, as shown in Fig. 1, following delivery of a stimulation pulse. Said parameters include maximum signal amplitude and maximum signal slope of the sensed IEGM signal, and area obtained by integrating the sensed IEGM signal over the evoked response detection time window. Further, the medical implant comprises a setting means 2.4 for setting a minimum tolerable difference between values of said selected parameter obtained in case of capture and in case of loss of capture respectively. The setting means 2.4 is adapted to set said minimum tolerable difference with a safety margin. The minimum tolerable difference is pre-set or programmable. The setting means 2.4 is adapted to calculate as said difference the signal-to-noise-ratio SNR from above-mentioned Equation [1]. A differentiating means 2.5 arranged to calculate the derivative of the sensed IEGM signal for the determination of the maximum slope, an integrating means 2.6 arranged to integrate the sensed IEGM signal over the evoked response detection window providing above-said area, and a maximum signal amplitude means 2.7 arranged to provide the maximum signal amplitude are provided. A first calculation means 2.8 is provided arranged to calculate for each of said parameters the length of the evoked response detection time window for which said minimum tolerable difference is obtained, together with a first selecting means 2.9 arranged to select that parameter for distinguishing capture and loss of capture for which said minimum tolerable difference is obtained with the shortest evoked response detection time window. Further, a third calculation means 2.10 is provided to calculate a matrix or table of said difference for different lengths of the evoked response detection time window and different ones of said parameters for storage for use in later off-line analysis.

Fig. 3 illustrates schematically a second preferred embodiment of the medical implant according to the present invention. As the embodiment of Fig. 2, this embodiment also comprises a pulse generator 3.2, an evoked response detector 3.3, a setting means 3.4, a differentiating means 3.5, an integrating means 3.6, and a maximum signal amplitude means 3.7, each with the same function as in the embodiment of Fig. 2. In this embodiment the evoked response detection time window has a fixed length. A second calculation means 3.8 is provided to calculate for each of said parameters the difference between the value of the parameter obtained in case of capture and in case of loss of capture respectively, and the second calculation means 3.8 is adapted to calculate as said difference the signal-to-noise-ratio SNR from above-mentioned Equation [1]. Further, a second selecting means 3.9 is provided to select that parameter for distinguishing capture and loss of capture by comparison with said minimum tolerable difference for which a maximum difference is obtained.

Fig. 4 shows a flow diagram illustrating a procedure performed by the above-mentioned first preferred embodiment of the medical implant according to the present invention, where the procedure includes the following steps:

4.1 Delivering a series of stimulation pulses to at least one chamber of a patient's heart, the amplitude of which ranging from zero to a certain maximum amplitude.

4.2 Recording the electrical activity in an evoked response time window of a certain maximum length after each stimulation pulse. The recording is performed by way of a modified VARIO test from the maximum amplitude down to zero without interrupting it.

4.3 Emitting a backup pulse at the end of the evoked response time window after each stimulation pulse.

4.4 Storing the electrical activity in an evoked response time window of a certain maximum length.

4.5 After completion of the recording, calculating the parameters for the evoked response time window of said certain maximum length from the stored values.

4.6 Determining the stimulation threshold for capture.

4.7 Calculating the signal-to-noise-ratio SNR from the above-mentioned Equation [1] for all parameters and multiple evoked response time window lengths.

4.8 Selecting that parameter for distinguishing capture from loss of capture for which the SNR is above a pre-set minimum tolerable difference with the shortest evoked response detection time window.

Fig. 5 shows a flow diagram illustrating a procedure performed by the second preferred embodiment of the medical implant according to the present invention. The length of the evoked response detection window is specified and fixed, and the procedure includes the steps 5.1 to 5.7, which correspond to the steps 4.1 to 4.7 of the procedure of Fig. 4, and step 5.8, which involves selecting that parameter for distinguishing capture and loss of capture by comparison with said minimum tolerable difference for which the greatest SNR is obtained is done.

## Claims

1. A medical implant comprising pulse generator means (2.2) for delivering stimulation pulses to at least one chamber of a patient's heart (2.1), an evoked response detector (2.3) for distinguishing capture from loss of capture from the value of a selected one of a plurality of parameters obtainable from an IEGM signal sensed in an evoked response detection time window following delivery of a stimulation pulse, and a setting means (2.4) for setting a minimum tolerable difference between values of said selected parameter obtained in case of capture and in case of loss of capture respectively, **characterized in that** the medical implant further comprises a first calculation means (2.8) to calculate for each of said parameters the length of the evoked response detection time window for which said minimum tolerable difference is obtained, and a first selecting means (2.9) to select that parameter for distinguishing capture and loss of capture for which said minimum tolerable difference is obtained with the shortest evoked response detection time window.

2. The medical implant according to claims 1, **characterized in that** said setting means (2.4) is adapted to set said minimum tolerable difference with a safety margin.

3. The medical implant according to claim 1, **characterized in that** a third calculation means (2.10) is provided to calculate a matrix or table of said difference for different lengths of the evoked response detection time window and different ones of said parameters for storage for use in later off-line analysis.

4. The medical implant according to any of the preceding claims, **characterized in that** said minimum tolerable difference is pre-set.

5. The medical implant according to any of the preceding claims, **characterized in that** said minimum tolerable difference is programmable.

6. The medical implant according to any of the preceding claims, **characterized in that** said setting means (2.4) is adapted to calculate as said difference the signal-to-noise-ratio SNR from the equation

$$\mathrm{SNR}=\left\{\begin{array}{l}\dfrac{\min(ERM_{\mathrm{capture}})-\max(ERM_{\mathrm{loss\,of\,capture}})}{\max(ERM_{\mathrm{capture}})-\min(ERM_{\mathrm{loss\,of\,capture}})},\ \overline{ERM}_{\mathrm{capture}}>\overline{ERM}_{\mathrm{loss\,of\,capture}}\\ \\ \dfrac{\max(ERM_{\mathrm{capture}})-\min(ERM_{\mathrm{loss\,of\,capture}})}{\min(ERM_{\mathrm{capture}})-\max(ERM_{\mathrm{loss\,of\,capture}})},\ \overline{ERM}_{\mathrm{capture}}<\overline{ERM}_{\mathrm{loss\,of\,capture}}\end{array}\right\}$$

$$\overline{ERM}_{\mathrm{capture}}\equiv\frac{1}{N}\sum_{i=1}^{N}ERM_{\mathrm{capture}}(i)$$

where $ERM_{capture}$ and $ERM_{loss\ of\ capture}$ denote the parameter values obtained in case of capture and loss of capture respectively.

7. The medical implant according to any of the preceding claims, **characterized in that** said parameters include maximum signal amplitude and maximum signal slope of the sensed IEGM signal, and area obtained by integrating the sensed IEGM signal over the evoked response detection time window.

8. The medical implant according to any of the preceding claims, **characterized in that** a differentiating means (2.5, 3.5) is provided to calculate the derivative of the sensed IEGM signal for the determination of the maximum slope.

9. The medical implant according to any of the preceding claims, **characterized in that** said pulse generator means (2.2) are controlled to deliver a stimulation back-up pulse at the end of the evoked response detection time window in response to detected loss of capture.

**Patentansprüche**

1. Medizinisches Implantat, enthaltend eine Impulsgeneratorvorrichtung (2.2) zum Liefern von Stimulationsimpulsen zu wenigstens einer Kammer des Herzens (2.1) eines Patienten, einen Detektor (2.3) für eine evozierte Reaktion zum Unterscheiden eines Captures von einem Capture-Verlust aus dem Wert eines ausgewählten Parameters aus einer Vielzahl von Parametern, die aus einem IEGM-Signal erhältlich sind, welches in einem Detektionszeitfenster der evozierten Reaktion abgefühlt worden ist, das auf die Ausgabe eines Stimulationsimpulses folgt, und eine Einstellvorrichtung (2.4) zum Einstellen einer minimalen tolerierbaren Differenz zwischen den Werten des genannten ausgewählten Parameters, die im Falle eines Captures bzw. im Falle eines Capture-Verlustes erhalten worden sind, **dadurch gekennzeichnet, dass** das medizinische Implantat ferner eine erste Berechnungsvorrichtung (2.8) enthält, um für jeden der genannten Parameter die Länge des Detektionszeitfensters der evozierten Reaktion zu berechnen, für das die genannte minimale tolerierbare Differenz erhalten wird, und eine erste Auswahlvorrichtung (2.9), um den Parameter zum Unterscheiden von Capture und Capture-Verlust auszuwählen, für den die genannte minimale tolerierbare Differenz mit dem kürzesten Detektionszeitfenster der evozierten Reaktion erhalten worden ist.

2. Medizinisches Implantat nach Anspruch 1, **dadurch gekennzeichnet, dass** die Einstellvorrichtung (2.4) ausgelegt ist, die genannte minimale tolerierbare Differenz mit einer Sicherheitsspanne einzustellen.

3. Medizinisches Implantat nach Anspruch 1, **dadurch gekennzeichnet, dass** eine dritte Berechnungsvorrichtung (2.10) vorgesehen ist, um zur Speicherung für die Verwendung bei einer späteren Offline-Analyse eine Matrix oder eine Tabelle der genannten Differenz für verschiedene Längen des Detektionszeitfensters der evozierten Reaktion und verschiedene der genannten Parameter zu berechnen.

4. Medizinisches Implantat nach einem der vorhergehenden Ansprüche, **dadurch gekennzeichnet, dass** die genannte minimale tolerierbare Differenz voreingestellt ist.

5. Medizinisches Implantat nach einem der vorhergehenden Ansprüche, **dadurch gekennzeichnet, dass** die genannte minimale tolerierbare Differenz programmierbar ist.

6. Medizinisches Implantat nach einem der vorhergehenden Ansprüche, **dadurch gekennzeichnet, dass** die genannte Einstellvorrichtung (2.4) ausgelegt ist als die genannte Differenz das Signal-Rausch-Verhältnis SNR aus der Gleichung

$$SNR = \begin{cases} \dfrac{\min(ERM_{capture}) - \max(ERM_{loss\ of\ capture})}{\max(ERM_{capture}) - \min(ERM_{loss\ of\ capture})}, \overline{ERM}_{capture} > \overline{ERM}_{loss\ of\ capture} \\ \\ \dfrac{\max(ERM_{capture}) - \min(ERM_{loss\ of\ capture})}{\min(ERM_{capture}) - \max(ERM_{loss\ of\ capture})}, \overline{ERM}_{capture} < \overline{ERM}_{loss\ of\ capture} \end{cases}$$

$$\overline{ERM}_{capture} \equiv \frac{1}{N}\sum_{i=1}^{N} ERM_{capture}(i)$$

zu berechnen, wobei $ERM_{capture}$ und $ERM_{loss\ of\ capture}$ die Parameterwerte bezeichnen, die im Falle eines Captures bzw. eines Capture-Verlustes erhalten werden.

7. Medizinisches Implantat nach einem der vorhergehenden Ansprüche, **dadurch gekennzeichnet, dass** die genannten Parameter eine maximale Signalamplitude und eine maximale Signalflanke des abgefühlten IEGM-Signals umfassen sowie einen durch Integrieren des abgefühlten IEGM-Signals über dem Detektionszeitfenster der evozierten Reaktion erhaltenen Bereich.

8. Medizinisches Implantat nach einem der vorhergehenden Ansprüche, **dadurch gekennzeichnet, dass** eine Differenziervorrichtung (2.5, 3.5) vorgesehen ist, um die Ableitung des abgefühlten IEGM-Signals zur Bestimmung der maximalen Flanke zu berechnen.

9. Medizinisches Implantat nach einem der vorhergehenden Ansprüche, **dadurch gekennzeichnet, dass** die genannte Impulsgeneratorvorrichtung (2.2) so gesteuert wird, dass sie in Antwort auf einen detektierten Capture-Verlust einen Stimulations-Sicherheitsimpuls am Ende des Detektionszeitfensters der evozierten Reaktion liefert.

## Revendications

1. Un implant médical comprenant un moyen (2.2) de générateur d'impulsions, pour envoyer des impulsions de stimulation à au moins une chambre d'un coeur de patient: (2.1), un détecteur (2.3) de réponse évoquée, pour distinguer une capture d'une perte de capture par la valeur d'un paramètre sélectionné parmi une pluralité de paramètres qui peuvent être obtenus à partir d'un signal d'IEGM détecté dans une fenêtre de temps de détection de réponse évoquée suivant l'envoi d'une impulsion de stimulation et un moyen (2.4) de réglage, pour régler une différence minimum acceptable entre des valeurs du dit paramètre sélectionné obtenu dans le cas d'une capture et dans le cas d'une perte de capture respectivement, **caractérisé en ce que** l'implant médical comprend en outre un premier moyen (2.8) de calcul, pour calculer pour chacun des paramètres, la longueur de la fenêtre de temps de détection de réponse évoquée, pour laquelle ladite différence minimum acceptable est obtenue, et un premier moyen (2.9) de sélection, pour sélectionner le paramètre de distinction de la capture et de la perte de capture, pour lequel la différence minimum acceptable est obtenue avec la fenêtre de temps de détection de réponse évoquée la plus courte.

2. L'implant médical suivant l'une quelconque des revendications 1, **caractérisé en ce que** le moyen (2.4) de réglage est conçu pour régler la différence minimum acceptable avec une marge de sécurité.

3. L'implant médical suivant la revendication 1, **caractérisé en ce qu'**il est prévu un troisième moyen (2.10) de calcul, pour calculer une matrice ou une table de ladite différence, pour des longueurs différentes de la fenêtre de temps de détection de réponse évoquée et différents paramètres parmi lesdits paramètres, en vue d'une mémorisation pour une utilisation dans une analyse ultérieure en discontinu.

4. L'implant médical suivant l'une quelconque des revendications précédentes, **caractérisé en ce que** la différence minimum acceptable est réglée à l'avance.

5. L'implant médical suivant l'une quelconque des revendications précédentes, **caractérisé en ce que** la différence minimum acceptable est programmable.

6. L'implant médical suivant l'une quelconque des revendications précédentes, **caractérisé en ce que** le moyen (2.4) de réglage est conçu pour calculer en tant que ladite différence le rapport SNR signal à bruit par l'équation

$$SNR = \begin{cases} \dfrac{\min(ERM_{capture}) - \max(ERM_{loss\ of\ capture})}{\max(ERM_{capture}) - \min(ERM_{loss\ of\ capture})}, & \overline{ERM}_{capture} > \overline{ERM}_{loss\ of\ capture} \\ \\ \dfrac{\max(ERM_{capture}) - \min(ERM_{loss\ of\ capture})}{\min(ERM_{capture}) - \max(ERM_{loss\ of\ capture})}, & \overline{ERM}_{capture} < \overline{ERM}_{loss\ of\ capture} \end{cases}$$

$$\overline{ERM}_{capture} = \frac{1}{N}\sum_{i=1}^{N} ERM_{capture}(i)$$

dans laquelle $ERM_{capture}$ et $ERM_{loss\ of\ capture}$ désignent les valeurs de paramètre obtenues dans le cas d'une capture et d'une perte de capture respectivement.

7. L'implant médical suivant l'une quelconque des revendications précédentes, **caractérisé en ce que** lesdits paramètres comprennent une amplitude maximum de signal et une pente maximum de signal du signal IEGM détecté, et des surfaces obtenues en intégrant le signal IEGM détecté sur la fenêtre de temps de détection.

8. L'implant médical suivant l'une quelconque des revendications précédentes, **caractérisé en ce qu'**il est prévu un moyen (2.5, 3.5) de différenciation, pour calculer la dérivé du signal IEGM détecté pour la détermination de la pente maximum.

9. L'implant médical suivant l'une quelconque des revendications précédentes, **caractérisé en ce que** ledit moyen (2.2) du générateur d'impulsions est commandé pour envoyer une impulsion de réserve à la fin de la fenêtre de temps de détection de réponse évoquée en réponse à une perte de capture détectée.

FIG. 1

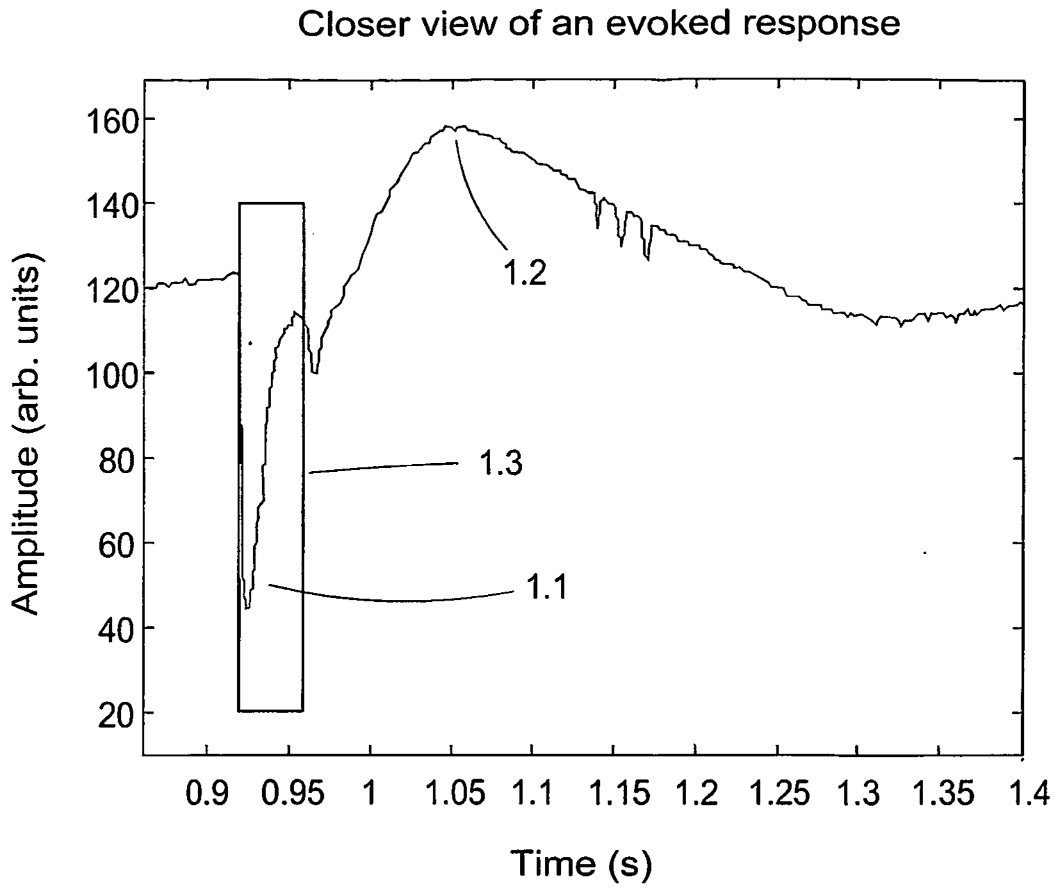
Closer view of an evoked response
Amplitude (arb. units)
160
140
120
100
80
60
40
20
0.9
0.95
1
1.05
1.1
1.15
1.2
1.25
1.3
1.35
1.4
Time (s)
1.1
1.2
1.3

FIG. 2

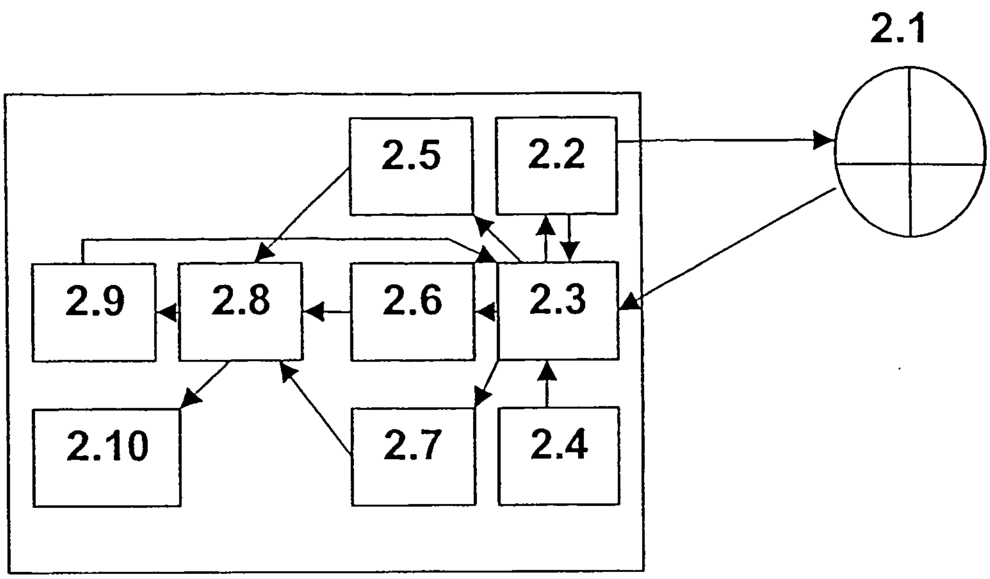

FIG. 3

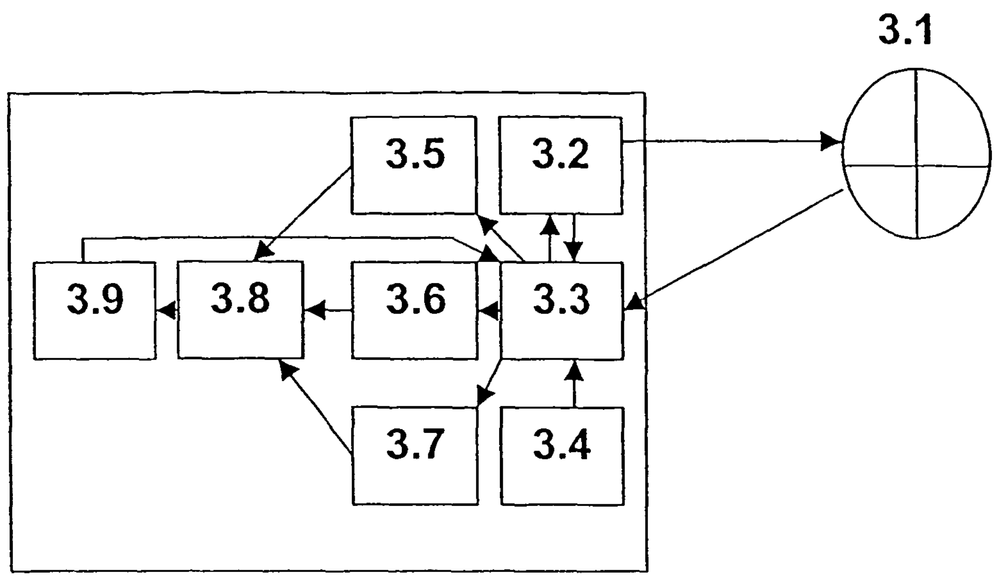

FIG 4

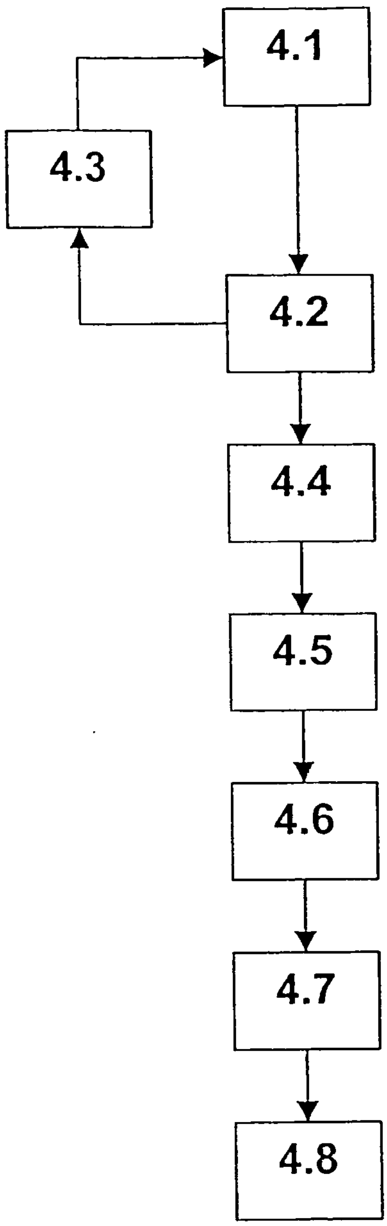

FIG 5

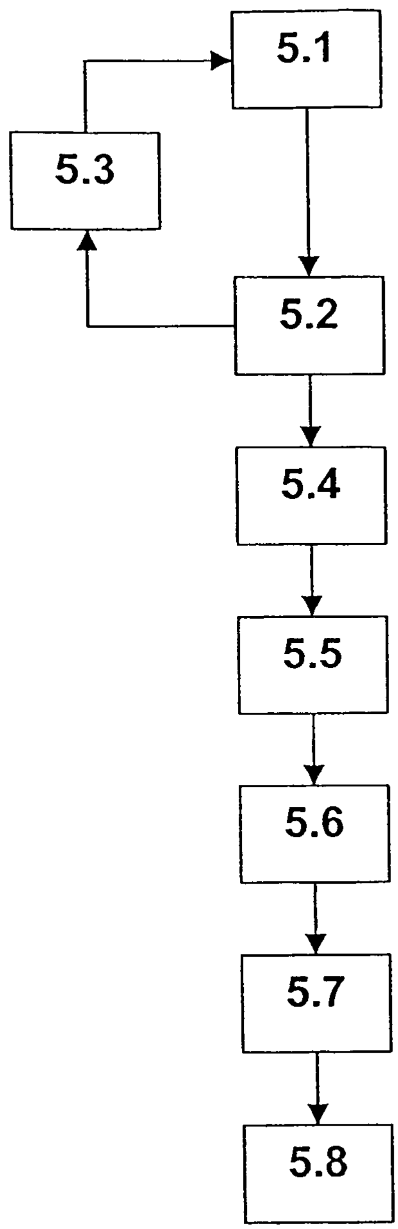

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5718720 A
- US 5855594 A

### Non-patent literature cited in the description

- Atrial Evoked Response Integral for Automatic Capture Verification In Atrial Pacing. *PACE 2003,* January 2003, vol. 26 (II), 1-5